# EUROPEAN PATENT SPECIFICATION

(11) **EP 0 956 811 B1**
(45) Date of publication and mention of the grant of the patent: **14.12.2005**
(21) Application number: 99201496.9
(22) Date of filing: 14.05.1999
(51) Int. Cl.: A61B 5/00

(54) **Device for monitoring and collecting data from mobile bodies**
Vorrichtung zur Datenüberwachung und -erfassung von bewegenden Körpern
Dispositif de surveillance et collecte de donneés de corps mobiles

(30) Priority: 15.05.1998 NL 1009175
(43) Date of publication of application: 17.11.1999
(73) Proprietor: K.U. LEUVEN RESEARCH & DEVELOPMENT, 3000 Leuven (BE)
(72) Inventor: Geers, Rony, Destelbergen (BE); Puers, Robert, Blanden (BE); Goedseels, Victor, 3010 Kessel-Lo (BE)
(74) Representative: Hoorweg, Petrus Nicolaas

(56) References cited:
- EP-A- 0 743 043
- WO-A-96/06409
- WO-A-97/12258
- GB-A- 2 258 589
- US-A- 5 487 760
- US-A- 5 499 626
- US-A- 5 526 772
- US-A- 5 588 234
- US-A- 5 626 630

## Description

The invention relates to a device for monitoring and collecting quantities of mobile bodies, wherein the device comprises:
- at least one detector placed in each of the bodies to be monitored for detecting a quantity to be monitored and for converting the quantity into an information signal;
- a memory for storing the information signals;
- a transmitter placed in each of the elements to be monitored for transmitting information signals; and
- a base station adapted to receive information signals.

Such a device is known from EP-A-0 549 081.

The device known from this literature reference is adapted to transfer data at regular times from the transmitter to the receiver, where the data is collected.

A drawback is that the periodic times at which the information transfer takes place cannot be fixed.

With the known information transfer a rather high transmission frequency must moreover be chosen in order to enable proper monitoring of rapid changes.

This means that the transmitter must be provided with a battery or storage battery for transmitting data, this for the entire lifespan of the transmitter. This results in a bulky and heavy device.

Another drawback is that the measuring characteristics cannot be modified to the individual application.

In the application a mobile body is understood to mean an independent mobile body, such as a live animal, as well as a body which is made mobile by an active intervention, for instance food products or animal or vegetable food products.

WO-A-97/12 258 discloses a system wherein the detector detects a quantity to be monitored (parameter) and converts the detected quantity (parameter value) into an information signal, that is stored in the memory. The information signal stored in the memory is only transmitted to the base station after receiving an interrogation signal.

However, the way in which the quantity is stored in memory is pre-preprogrammed. In other words, the recording protocol for recording the data measured by the detector is fixed.

GB-A-2 258 589 discloses a device including a transponder and a detector wherein the quantity to be monitored is recorded according to a recording protocol. The known device comprises a memory, which can be programmed once (one time programmable memory) and the memory is filled with identification codes for the animals being monitored. In other words, the memory can be programmed only in the sense that in the memory identification codes may be stored. The recording protocol of recording the quantity therefore is fixed as well.

It is an object of the present invention to provide a system and a device for monitoring and collecting quantities of mobile bodies wherein the above mentioned drawbacks are obviated.

This object is achieved in a system as claimed in claim 1, and a device as claimed in claim 19.

The base station is adapted to transmit interrogation signals and the transmitters placed in the bodies to be monitored are each formed by transponders which are adapted to receive the interrogation signals and to transmit information signals stored in the memory in response to receiving an interrogation signal.

As a result of these measures it is first of all possible to make use of a transponder.

Another advantage of the device according to the invention is the fact that the initiative for retrieving data lies with the bases station. This latter after all holds the information which has become available in the past, so that a development in the quantity at a higher frequency is registered, whereafter this information can be retrieved at shorter intervals, whereby such high-frequency phenomena can also be included in the monitoring.

A transponder has the advantage of a lower energy consumption, since the transmitting energy required to transmit the messages or data can be drawn from the energy received when messages are received, in the present case the interrogation message.

According to the present invention a transponder without battery can be coupled to an actively operating measuring transponder.

According to a preferred embodiment at least the transponder is adapted to receive and transmit signals in the ether.

As a result of these measures it is possible for the object to be monitored to move freely not just during the information exchange but continuously; if use were made of a galvanic connection, it would be necessary to effect a galvanic connection each time information exchange took place.

A particularly attractive embodiment is created when at least the base station is adapted to receive and transmit signals via a telephone network. This provides the option of placing the base station a great distance from the objects to be monitored.

According to yet another preferred embodiment the transponder is adapted for information communication via a mobile telephone network.

This feature provides the advantage that use can be made of the mobility of mobile telephone networks; it is possible to embody a transponder so that it can communicate with a mobile telephone network, this having the advantage that lower investment costs will suffice to obtain mobility; use can after all be made of said facilities here.

The present invention also relates to such a transponder, a food chain product provided with such a transponder and a method for monitoring and collecting quantities of mobile bodies, comprising the steps of:
- detecting a quantity to be monitored and converting the quantity into an information signal;
- storing the information signals in a memory;
- transmitting information signals; and
- receiving information signals, wherein only on reception of an interrogation signal a response thereto is transmitted of an information signal stored in a memory.

The invention allows wireless programming of a recording protocol. This enables a telemetric variation of the recording protocol.

Examples of interrogated variables are temperature, activity, moisture, pH, pressure and other chemical and physical variables. An additional advantage is that the programming of the data reception by the transponder as well as the calibration of the sensors by the RF-link, also when the transponder is already operational, is possible so that the transponder is adjustable to each new situation.

An other advantage of the invention is the possibility to make a link and correlation between an electronic identification number and a genetical characteristic, for example a DNA-characteristic of the movable body. Because of this fraude is further complicated.

Further additional advantages are:
- that the process qualification/identification of logistic/production processes and chains are carried out;
- that several process stages can be distinguished by a code and can therefore be used as onus of proof;
- that specific features and particulars keep staying present with the movable body during the whole cycle.

From the above mentioned it is clear that the device according to the invention in particular makes fraude and deceit impossible due to the possibility of maximalizing interrogation in time and space and on the other hand by an enforced individual identification.

## Claims

1. System for monitoring and collecting quantities of mobile bodies, wherein the system comprises:
- a base station adapted to receive information signals and to transmit interrogation signals;
- at least one device placed in each of the bodies to be monitored, the device comprising:
- a detector for recording a quantity to be monitored according to a recording protocol and converting the quantity into an information signal;
- a memory for storing the information signals;
- a transponder disposed in each of the bodies to be monitored, the transponders being adapted to receive the interrogation signals and to transmit the information signals stored in the memory in response to receiving an interrogation signal,
**characterized in that**
the device is adapted to allow wireless programming of said recording protocol.

2. System as claimed in claim 1, wherein the wireless programming of the recording protocol comprises a telemetric variation of the recording protocol.

3. System according to claim 1 or 2, wherein said device is adapted so as to allow wireless calibration of the detector placed in each of te bodies to be monitored.

4. System according to claim 1, 2 or 3, wherein the wireless programming and/ore the wireless calibration is possible when the device is operational.

5. System according to any of the preceding claims, wherein the base station and the device are adapted to receive and transmit signals in the ether so as to wirelessly program the recording protocol of the device.

6. System according to any of the claims 1-5, wherein the transponder is configured so as to draw the transmitting energy required to transmit the information signals to the base station from the energy received when the interrogation signals are received.

7. System as claimed in any of the claims 1-6, wherein the transponder is adapted to receive and transmit signals via a telephone network.

8. Systems as claimed in claim 7, wherein the transponder is adapted for information communication via a mobile telephone network.

9. System according to any of the preceding claims, wherein the mobile body is adapted as a food chain product.

10. System according to claim 9, wherein the food chain product is of an animal or vegetable origin.

11. System according to claim 10, wherein the food chain product is a live animal.

12. System according to any of the preceding claims **characterized in that** the quantities monitored by the system are chosen from the set comprising identification, temperature, activity, moisture, and/or pH or pressure.

13. System according to claim 11 **characterized in that** the system allows the monitoring of the reproduction cycle of an animal.

14. System according to claim 9 **characterized in that** the system is capable of monitoring parameter quantities associated with the storage conditions of a food chain product.

15. System according to claim 9 **characterized in that** the systems allows the identification of the origin of a food chain product.

16. System according to claim 15 **characterized in that** the identification of the origin of a food chain product is coupled to a DNA-characteristic of that product.

17. System according to any of the preceding claims **characterized in that** the system allows the qualification/identification of logistic/production processes and chains are carried out.

18. System according to any of the preceding claims **characterized in that** the system attributes to different process stages a code and whereby said code can be used as onus of proof.

19. A device for monitoring and collecting a quantity associated with a mobile body, comprising:
- a detector disposed within the mobile body and configured to record the quantity according to a recording protocol and generate an information signal representing the quantity;
- a memory coupled to the detector and configured to store data representing the information signal; and
- a transponder coupled to the memory and configured to transmit the data in response to the receiving of an interrogation signal,
**characterized in that**
the device is adapted so as to allow wireless programming of said recording protocol.

20. Device according to claim 19, wherein the wireless programming of the recording protocol comprises the telemetric variation of the recording protocol.

21. Device according to claim 19 or 20, wherein said device is adapted so as to allow wireless calibration of the detector placed in each of the bodies to be monitored.

22. Device according to claim 20 or 21, wherein the wireless programming and/ore the wireless calibration is possible when the device is operational.

23. Device according to any of the claims 19-22, wherein the device is adapted to receive signals in the ether from a base station so as to wirelessly program the recording protocol of the device by the base station.

24. Device according to any of claims 19-23, wherein the transponder is configured so as to draw the transmitting energy required to transmit the information signals to the base station from the energy received when the interrogation signals are received.

25. Device as claimed in any of claims 19-24, wherein at least the device is adapted to receive and transmit signals in the ether so as to wirelessly program the recording protocol of the device.

26. Device as claimed in claim 25, wherein the transponder is adapted to receive and transmit signals via a telephone network.

27. Device as claimed in claim 26, wherein the transponder is adapted for information communication via a mobile telephone network.

## Patentansprüche

1. System zur Überwachung und zum Sammeln von Quantitäten von mobilen Körpern, wobei das System aufweist:
- eine Basisstation, die Informationssignale empfangen und Abfragesignale senden kann;
- wenigstens eine Vorrichtung, die in jedem der zu überwachenden Körper platziert ist, wobei die Vorrichtung aufweist:
- einen Detektor zum Aufzeichnen einer zu überwachenden Quantität gemäß einem Aufzeichnungsprotokoll und Umwandeln der Quantität in ein Infor mationssignal;
- einen Speicher zum Speichern der Informationssignale;
- einen Transponder, der in jedem der zu überwachenden Körper angeordnet ist, wobei der Transponder so ausgebildet ist, dass er die Abfragesig nale empfangen kann und die Informationssignale, die in dem Speicher gespeichert sind, in Antwort auf das Empfangen eines Abfragesignals übertragen kann,
**dadurch gekennzeichnet, dass**
die Vorrichtung so ausgebildet ist, dass sie eine drahtlose Programmierung des Aufzeichnungsprotokolls ermöglicht.

2. System nach Anspruch 1, wobei die drahtlose Programmierung des Aufzeichnungsprotokolls eine telemetrische Änderung des Aufzeichnungsprotokolls umfasst.

3. System nach Anspruch 1 oder 2, wobei die Vorrichtung so ausgebildet ist, dass sie eine drahtlose Kalibrierung des Detektors ermöglicht, der in jedem der zu überwachenden Körper platziert ist.

4. System nach Anspruch 1, 2 oder 3, wobei die drahtlose Programmierung und/oder die drahtlose Kalibrierung möglich ist, wenn die Vorrichtung im Betrieb ist.

5. System nach einem der vorstehenden Ansprüche, wobei die Basisstation und die Vorrichtung so ausgebildet sind, dass sie Signale aus dem Äther empfangen und in den Äther übertragen können, um das Aufzeichnungsprotokoll der Vorrichtung drahtlos zu programmieren.

6. System nach einem der Ansprüche 1 bis 5, wobei der Transponder so konfiguriert ist, dass er die Sendeenergie, die für das Übertragen der Informationssignale auf die Basisstation erforderlich ist, aus der Energie zieht, die empfangen wird, wenn die Abfragesignale empfangen werden.

7. System nach einem der Ansprüche 1 bis 6, wobei der Transponder so ausgebildet ist, dass er Signale über ein Telefonnetzwerk empfangen und senden kann.

8. System nach Anspruch 7, wobei der Transponder für die Informationskommunikation über eine mobiles Telefonnetz ausgerüstet ist.

9. System nach einem der vorstehenden Ansprüche, wobei der mobile Körper als ein Nahrungsmittelkettenprodukt ausgebildet ist.

10. System nach Anspruch 9, wobei das Nahrungsmittelkettenprodukt tierischen oder pfanzlichen Ursprungs ist.

11. System nach Anspruch 10, wobei das Nahrungsmittelkettenprodukt ein lebendes Tier ist.

12. System nach einem der vorstehenden Ansprüche, **dadurch gekennzeichnet, dass** die durch das System zu überwachenden Quantitäten aus dem Satz gewählt werden, der besteht aus Identifikation, Temperatur, Aktivität, Feuchtigkeit und/oder pH-Wert oder Druck.

13. System nach Anspruch 11, **dadurch gekennzeichnet, dass** das System die Überwachung des Reproduktionszyklus eines Tieres erlaubt.

14. System nach Anspruch 9, **dadurch gekennzeichnet, dass** das System Parameterquantitäten, die den Lagerbedingungen für ein Nahrungsmittelkettenprodukt zugeordnet sind, überwachen kann.

15. System nach Anspruch 9, **dadurch gekennzeichnet, dass** die Systeme die Identifikation des Ursprungs eines Nahrungsmittelkettenproduktes ermöglichen.

16. System nach Anspruch 15, **dadurch gekennzeichnet, dass** die Identifikation des Ursprungs eines Nahrungsmittelkettenproduktes an eine DNA-Charakteristik dieses Produktes gekoppelt ist.

17. System nach einem der vorstehenden Ansprüche, **dadurch gekennzeichnet, dass** das System die Qualifikation/Identifikation von logistischen/Produktionsprozessen erlaubt und Ketten ausgeführt werden.

18. System nach einem der vorstehenden Ansprüche, **dadurch gekennzeichnet, dass** das System unterschiedlichen Prozessstufen einen Kode zuschreibt und wodurch dieser Kode als Beweislast verwendet werden kann.

19. Vorrichtung zur Überwachung und zum Sammeln einer Quantität, zugeordnet zu einem mobilen Körper, mit:
- einem Detektor, der innerhalb des mobilen Körpers angeordnet ist und so konfiguriert ist, dass er die Quantität gemäß einem Aufzeichnungsprotokoll aufzeichnen und ein Informationssignal erzeugen kann, das die Quantität repräsentiert;
- einem Speicher, der an den Detektor gekoppelt ist und so konfiguriert ist, dass er Daten, welche das Informationssignal repräsentieren, speichern kann; und
- einem Transponder, der an den Speicher gekoppelt ist und so konfiguriert ist, dass er Daten in Antwort auf den Empfang eines Anfragesignals übertragen kann,
**dadurch gekennzeichnet, dass**
die Vorrichtung so ausgebildet ist, dass sie eine drahtlose Programmierung des Aufzeichnungsprotokolls zulässt.

20. Vorrichtung nach Anspruch 19, wobei die drahtlose Programmierung des Aufzeichnungsprotokolls die telemetrische Änderung des Aufzeichnungsprotokolls umfasst.

21. Vorrichtung nach Anspruch 19 oder 20, wobei die Vorrichtung so ausgebildet ist, dass sie eine drahtlose Kalibrierung des in jedem zu überwachenden Körper platzierten Detektors ermöglicht.

22. Vorrichtung nach Anspruch 20 oder 21, wobei die drahtlose Programmierung und/oder die drahtlose Kalibrierung bei im Betrieb befindlicher Vorrichtung möglich ist.

23. Vorrichtung nach einem der Ansprüche 19 bis 22, wobei die Vorrichtung so ausgebildet ist, dass sie Signale aus dem Äther von einer Basisstation empfangen kann, um das Aufzeichnungsprotokoll der Vorrichtung durch die Basisstation drahtlos zu programmieren.

24. Vorrichtung nach einem der Ansprüche 19 bis 23, wobei der Transponder so konfiguriert ist, dass er die Sendeenergie, die für das Übertragen der Informationssignale auf die Basisstation erforderlich ist, aus der Energie ziehen kann, die empfangen wird, wenn die Anfragesignale empfangen werden.

25. Vorrichtung nach einem der Ansprüche 19 bis 24, wobei wenigstens die Vorrichtung so ausgebildet ist, dass sie Signale aus dem Äther empfangen und in den Äther übertragen kann, um das Aufzeichnungsprotokoll der Vorrichtung drahtlos zu programmieren.

26. Vorrichtung nach Anspruch 25, wobei der Transponder so ausgebildet ist, dass er Signale über ein Telefonnetzwerk empfangen und übertragen kann.

27. Vorrichtung nach Anspruch 26, wobei der Transponder für die Informationskommunikation über ein Mobiltelefonnetzwerk ausgebildet ist.

## Revendications

1. Système pour surveiller et collecter des quantités de corps mobiles, dans lequel le système comprend :
- une station de base adaptée pour recevoir des signaux d'information et pour transmettre des signaux d'interrogation ;
- au moins un dispositif placé dans chacun des corps devant être surveillé, le dispositif comprenant :
- un détecteur pour enregistrer une quantité devant être surveillée selon un protocole d'enregistrement et convertir la quantité en un signal d'information ;
- une mémoire pour emmagasiner les signaux d'information ;
- un transpondeur disposé dans chacun des corps devant être surveillés, les transpondeurs étant adaptés pour recevoir les signaux d'interrogation et pour transmettre les signaux d'informations emmagasinés dans la mémoire en réponse à la réception d'un signal d'interrogation,
**caractérisé en ce que**
le dispositif est adapté pour permettre une programmation sans fil dudit protocole d'enregistrement.

2. Système selon la revendication 1, dans lequel la programmation sans fil du protocole d'enregistrement comprend une variation télémétrique du protocole d'enregistrement.

3. Système selon la revendication 1 ou 2, dans lequel ledit dispositif est adapté afin de permettre un étalonnage sans fil du détecteur placé dans chacun des corps devant être surveillés.

4. Système selon la revendication 1, 2 ou 3, dans lequel la programmation sans fil et/ou l'étalonnage sans fil est possible lorsque le dispositif est en état de fonctionnement.

5. Système selon l'une quelconque des revendications précédentes, dans lequel la station de base et le dispositif sont adaptés pour recevoir et transmettre les signaux vers l'espace afin de programmer sans fil le protocole d'enregistrement du dispositif.

6. Système selon l'une quelconque des revendications 1 à 5, dans lequel le transpondeur est configuré de façon à tirer l'énergie de transmission nécessaire pour transmettre les signaux d'information vers la station de base depuis l'énergie reçue lorsque les signaux d'interrogation sont reçus.

7. Système selon l'une quelconque des revendications 1 à 6, dans lequel le transpondeur est adapté pour recevoir et transmettre des signaux par le biais d'un réseau téléphonique.

8. Systèmes selon la revendication 7, dans lesquels le transpondeur est adapté pour une communication d'informations par l'intermédiaire d'un réseau téléphonique mobile.

9. Système selon l'une quelconque des revendications précédentes, dans lequel le corps mobile est adapté comme produit de chaîne alimentaire.

10. Système selon la revendication 9, dans lequel le produit de chaîne alimentaire est d'origine animale ou végétale.

11. Système selon la revendication 10, dans lequel le produit de chaîne alimentaire est un animal vivant.

12. Système selon l'une quelconque des revendications précédentes, **caractérisé en ce que** les quantités surveillées par le système sont choisies à partir de l'ensemble comprenant une identification, une température, une activité, une humidité, et/ou un pH ou une pression.

13. Système selon la revendication 11, **caractérisé en ce que** le système permet la surveillance du cycle de reproduction d'un animal.

14. Système selon la revendication 9, **caractérisé en ce que** le système est capable de surveiller des quantités de paramètres associées aux conditions de stockage d'un produit de chaîne alimentaire.

15. Système selon la revendication 9, **caractérisé en ce que** les systèmes permettent l'identification de l'origine d'un produit de chaîne alimentaire.

16. Système selon la revendication 15, **caractérisé en ce que** l'identification de l'origine d'un produit de chaîne alimentaire est couplée à une caractéristique d'ADN de ce produit.

17. Système selon l'une quelconque des revendications précédentes, **caractérisé en ce que** le système permet la qualification/identification de processus logistique/de production et des chaînes sont mises en oeuvre.

18. Système selon l'une quelconque des revendications précédentes, **caractérisé en ce que** le système attribue à différentes phases de processus un code et en conséquence de quoi, ledit code peut être utilisé en tant que charge de preuve.

19. Dispositif pour surveiller et collecter une quantité associée à un corps mobile, comprenant :
- un détecteur disposé à l'intérieur du corps mobile et configuré pour enregistrer la quantité selon un protocole d'enregistrement et générer un signal d'information représentant la quantité ;
- une mémoire couplée au détecteur et configurée pour emmagasiner des données représentant le signal d'information ; et
- un transpondeur couplé à la mémoire et configuré pour transmettre les données en réponse à la réception d'un signal d'interrogation,
**caractérisé en ce que**
le dispositif est adapté de façon à permettre une programmation sans fil dudit protocole d'enregistrement.

20. Dispositif selon la revendication 19, dans lequel la programmation sans fil du protocole d'enregistrement comprend la variation télémétrique du protocole d'enregistrement.

21. Dispositif selon la revendication 19 ou 20, dans lequel ledit dispositif est adapté afin de permettre un étalonnage sans fil du détecteur placé dans chacun des corps devant être surveillé.

22. Dispositif selon la revendication 20 ou 21, dans lequel la programmation sans fil et/ou l'étalonnage sans fil est possible lorsque le dispositif est en état de fonctionnement.

23. Dispositif selon l'une quelconque des revendications 19 à 22, dans lequel le dispositif est adapté pour recevoir des signaux dans l'espace depuis une station de base afin de programmer sans fil le protocole d'enregistrement du dispositif grâce à la station de base.

24. Dispositif selon l'une quelconque des revendications 19 à 23, dans lequel le transpondeur est configuré de façon à tirer l'énergie de transmission requise pour transmettre les signaux d'informations vers la station de base depuis l'énergie reçue lorsque les signaux d'interrogation sont reçus.

25. Dispositif selon l'une quelconque des revendications 19 à 24, dans lequel au moins le dispositif est adapté pour recevoir et transmettre des signaux dans l'espace afin de programmer sans fil le protocole d'enregistrement du dispositif.

26. Dispositif selon la revendication 25, dans lequel le transpondeur est adapté pour recevoir et transmettre des signaux par le biais d'un réseau téléphonique.

27. Dispositif selon la revendication 26, dans lequel le transpondeur est adapté pour une communication d'informations par l'intermédiaire d'un réseau téléphonique mobile.
